(19) Europäisches Patentamt
European Patent Office
Office européen des brevets

(11) Numéro de publication: **0 050 070**
**B1**

# (12) FASCICULE DE BREVET EUROPEEN

(45) Date de publication du fascicule du brevet: **25.01.84**

(51) Int. Cl.³: **A 61 L 2/08**, C 08 L 27/06, C 08 K 5/00 // C08J7/10

(21) Numéro de dépôt: **81401511.1**

(22) Date de dépôt: **30.09.81**

(54) **Perfectionnement à la stérilisation d'objets en polymères halogéno-vinyliques par des rayonnements ionisants.**

(30) Priorité: **10.10.80 FR 8021662**

(43) Date de publication de la demande: **21.04.82 Bulletin 82/16**

(45) Mention de la délivrance du brevet: **25.01.84 Bulletin 84/4**

(84) Etats contractants désignés: **AT CH DE GB LI NL SE**

(56) Documents cités:
**EP-A-0 010 008**
**DE-A-1 217 609**
**GB-A-827 393**
**GB-A-1 178 739**

(73) Titulaire: **ATO CHIMIE, Société Anonyme dite:, La Défense 5 12/16 Allée des Vosges, F-92400 Courbevoie (FR)**

(72) Inventeur: **Kornbaum, Simon, 212, rue Benjamin Delessert, F-69300 Caluire (FR)**
Inventeur: **Chenard, Jean-Yves, 19 bis rue Bernes Cambot, F-64000 Pau (FR)**

(74) Mandataire: **Kohn, Armand, 5 Avenue Foch, F-92380 Garches (FR)**

**EP 0 050 070 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

# Perfectionnement à la stérilisation d'objets en polymères halogéno-vinyliques par des rayonnements ionisants

La présente invention concerne un perfectionnement au procédé de stérilisation à l'aide de rayonnements ionisants, appliqué à des objets en matière plastique à base de polymères dont les molécules renferment de l'halogène. Elle se rapporte en particulier aux articles en polymères d'halogénovinyle ou vinylidène, et plus spécialement à ceux de chlorure de polyvinyle et aux copolymères de ce dernier.

La stérilisation par irradiation avec des rayons ionisants, c'est-à-dire rayonnements de haute énergie, notamment rayons X, gamma, béta, etc. est connue: elle a été décrite par exemple dans les comptes rendus du Colloque de Deauville, du 10 Novembre 1977, organisé par SNPM Bio-Conseil Neuilly (article de MM. Icre et Vidal). Elle est fort utile, lorsqu'on veut assurer la bonne conservation d'un produit ou denrée dans un récipient; si le produit n'est pas altéré par une brève irradiation, on soumet le récipient fermé, qui le contient, aux rayons ionisants, capables de traverser les parois du récipient; il en résulte une stérilisation rapide, à cœur. Aucune contamination ne peut plus se produire par la suite, lorsque les parois du récipient sont imperméables aux microorganismes, ce qui est le cas des parois en polymères halogéno-vinyliques et similaires. Il faut, cependant, que la matière, constituant le récipient, reste inaltérée par ce traitement; or, à partir d'une certaine dose, nécessaire à la stérilisation, les rayonnements ionisants produisent des altérations dans la masse de nombreuses résines, en particulier dans celle du chlorure de polyvinyle et de ses copolymères, ce qui se manifeste par une coloration plus ou moins intense de la matière.

Ainsi, par exemple, des objets moulés, en chlorure de polyvinyle stabilisé à la chaleur par des adjuvants habituels, notamment des organo-stanniques et des thiocomposés, jaunissent dès que la dose de rayons gamma atteint environ 0,5 Mégarad; l'altération devient très marquée quand la dose avoisine 1 Mrad, et elle prend des proportions inacceptables pour le maximum de 2,76 Mrads, alors que la stérilisation peut exiger des doses de 1 à 2,76 Mrads.

Il n'est pas surprenant que les stabilisants à la chaleur habituels d'halogéno-résines, en particulier les composés organo-stanniques accompagnés de thiocomposés organiques, ne protègent pas ces résines contre l'action délétère des rayonnements ionisants, puisque cette dernière action est de nature différente de celle de la chaleur. Mais ce qui est étonnant c'est que la présence de certains mercaptans particuliers, conjointement avec d'autres stabilisants classiques, permette d'éviter l'altération des résines en question, pour des doses de rayonnement ionisant pouvant aller jusqu'aux environs de 2,8 Mrads. Et c'est sur cette constatation inattendue qu'est basée la présente invention.

L'invention présente l'important avantage de permettre la stabilisation aux rayonnements ionisants de polymères exempts de plastifiants. Or, il apparaît que, jusqu'à présent, seuls certains plastifiants permettraient d'améliorer la tenue du chlorure de polyvinyle vis-à-vis des rayons $\gamma$. Ainsi, relate-t-on, dans Chem. Abstr. vol. 61 (1964), 3263h, les publications de Tadashi Kimura (Osaka, Munic. Tech. Res. Inst. Japan), d'après lesquelles des plastifiants du type phosphates, le sébaçate ou le phtalate de bis (éthyl-2 hexyle) donneraient des résultats favorables à ce point de vue. Cependant, dans la pratique, on est souvent amené à soumettre au rayonnement ionisant une résine qui ne doit pas contenir de plastifiant. Un cas important est celui de la stérilisation de produits ou denrées contenus dans des flacons ou autres emballages en chlorure de polyvinyle: d'une part la bonne tenue mécanique des emballages exige une rigidité suffisante, donc absence de plastifiant; d'autre part, les plastifiants risquent de contaminer le produit emballé ce qui se traduirait, en particulier, par un danger sanitaire pour des produits alimentaires, cosmétiques, hygiéniques ou pharmaceutiques.

Suivant l'invention, un procédé perfectionné de stérilisation d'objets en matière plastique, dont la molécule renferme de l'halogène, consiste à incorporer à la matière plastique, renfermant un stabilisant classique, en particulier organostanique ou/et organo-antimoneux, un ou plusieurs esters porteurs de la fonction mercaptan-(-SH) à raison de 1 groupe -SH pour 3 à 10 atomes de carbone, et à soumettre l'objet, formé avec la matière obtenue, à un rayonnement ionisant de haute énergie, préférablement à la dose de 0,5 à 2,8 Mégarads.

Le polymère des objets ainsi stérilisés, reste incolore ou faiblement jaunâtre, s'il n'était pas coloré au départ. Il en résulte que les objets, obtenus par l'application du procédé de l'invention, sont caractérisés par la similitude de leur teinte avec celle du polymère utilisé.

Les résines, justiciables du nouveau procédé, sont constituées par les divers polymères dont la molécule possède des atomes d'halogène; ce sont, plus spécialement, des résines halogéno-vinyliques, dont les types industriellement les plus importants sont les polyéthylène chloré, polydichlorure de vinylidène, chlorure de polyvinyle, acétochlorure de polyvinyle, chloropolyéther, polychloro-trifluoroéthylène, poly-fluorovinylidène, fluorure de polyvinyle, les différents copolymères correspondants, notamment ceux du chlorure avec l'acétate de vinyle, chlorure de vinyle avec celui de vinylidène ou avec des oléfines, etc.

Les stabilisants à la chaleur classiques, dont la présence est nécessaire dans la résine devant être traitée suivant l'invention, sont connus dans l'art. On peut citer à titre d'exemples les composés

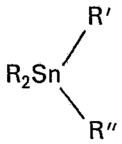 , 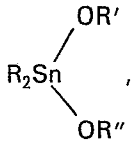 , 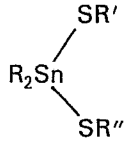 ,

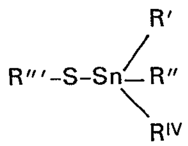 et

corps similaires, dans lesquels les symboles R, R′, R″, R‴, $R^{IV}$ désignent différents groupes organiques et éventuellement inorganiques. Tels sont notamment les composés:

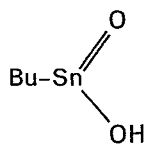 ; 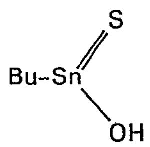 ;

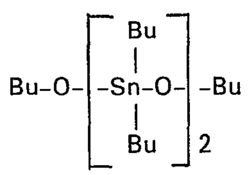 ;

$Bu_2Sn(OOC-C_{11}H_{23})_2$; 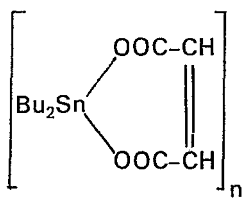 ;

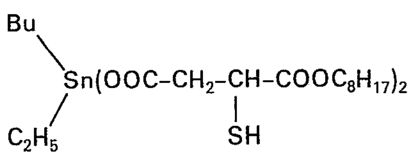

$(C_7H_{15})_2Sn(SC_8H_{17})_2$; $Bu_2Sn(OOC-CH_2SH)_2$;

$(C_{10}H_{21})_2Sn(OCH_3)_2$; $(C_8H_{17})_2Sn(SCH_2-COOC_8H_{17})_2$

Les composés stanniques peuvent être remplacés ou accompagnés par des combinaisons équivalentes de l'antimoine. D'autres composés métalliques peuvent également être employés.

Les proportions des stabilisants organo-métalliques peuvent être les mêmes que celles de la technique connue pour la stabilisation à la chaleur, c'est-à-dire généralement de 0,01 à 5% en poids de la résine, et le plus souvent de l'ordre de 0,5 à 2,5%. D'ailleurs, une façon pratique de réaliser l'invention consiste à se servir d'une résine prête à l'emploi, renfermant tous les additifs utiles, dont les stabilisants vis-à-vis de la chaleur, de la malaxer à chaud, à l'état semi fondu, pour y incorporer un ou plusieurs esters-thiols suivant l'invention, et de la mettre en la forme voulue, avant le traitement aux rayons ionisants: ainsi peut-on employer les compositions décrites dans le brevet français n° 1 440 656, notamment celles dans lesquelles l'étain est sous la forme de thioglycolate; conviennent également les compositions selon la publication française n° 2 434 835, les brevets U.S. n° 3 063 963 - 3 507 827 - 3 970 689 ou 2 914 506. Ces compositions subissent une altération nette dès que la dose de rayonnement dépasse 0,5 Mégarad, alors qu'elles suportent 1 Mégarad et plus quand on leur a incorporé au moins un des mercapto-esters suivant l'invention. Ce fait est d'autant plus inattendu que nombreuses des compositions connues renferment de l'acide thio-glycolique, libre ou combiné, qui est justement un mercaptan, $HS-CH_2COOH$: et pourtant elles ne résistent à des doses de 1 Mégarad ou plus que si on leur a incorporé un des esters mercaptans spéciaux suivant l'invention.

Comme indiqué plus haut, les esters mercaptans, ajoutés à la résine conformément à l'invention, renferment 1 fonction -SH pour 3 à 10 atomes de carbone. De préférence, la molécule de l'ester porte deux groupes -SH et il est avantageux que ces groupes se trouvent aux bouts opposés de la chaîne qui forme la molécule. A titre d'exemples non limitatifs, voici quelques-uns des esters mercaptans dérivés de monoacides carboxyliques, faisant l'objet de l'invention.

| | Rapport SH/C |
|---|---|
| thiolactate d'éthyle 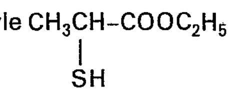 | 1/5 |
| mercapto-6 caproate de méthyle $HS(CH_2)_5COOCH_3$ | 1/6 |
| caproate de mercapto-2 éthyle $CH_3(CH_2)_4-COO-CH_2CH_2SH$ | 1/8 |
| mercapto-6 caproate de mercapto-2 éthyle $HS-(CH_2)_5-COO-CH_2CH_2SH$ | 1/4 |
| mercapto-12 laurate de mercapto-2 éthyle $HS-(CH_2)_{11}-COO-CH_2CH_2SH$ | 1/7 |

On peut noter à ce sujet que l'acide thioglycolique, si couramment employé selon les brevets cités plus haut, n'est pas un ester, et son rapport SH/C est 1/2; comme vu plus haut, il ne protège pas contre les rayonnements ionisants.

Selon une forme d'exécution de l'invention, particulièrement intéressante, l'ester mercaptan, incorporé à la résine, dérive d'un diacide carboxylique ou d'un polyol et porte deux groupes -SH, chacun à un des bouts opposés de la molécule. Ces esters mercaptans préférés peuvent être représentés par la formule générale:

(1) $HS-(CH_2)_n-Z-(CH_2)_m-Z-(CH_2)_n-SH$

où Z désigne le groupe carboxy $-\underset{\underset{O}{\|}}{C}-O-$ dont l'atome de C est lié à un atome de carbone des chaînes $(CH_2)_n$ ou de la chaîne $(CH_2)_m$. Le nombre entier n peut varier de 1 à 9 et m de 1 à 18, à la condition cependant que la somme 2n+m soit d'au moins 4.

Chacune des chaînes $-(CH_2)_n-$ et $-(CH_2)_m-$ peut d'ailleurs porter des substituants; des groupes -OH ou -SH y sont fort utiles.

De ce qui précède on voit que les fonctions mercaptan -SH peuvent se trouver dans le reste d'acide ou/et dans le reste d'alcool de l'ester; en effet, l'ester de formule (1) peut provenir de l'estérification d'un

- polyol $HO(CH_2)_mOH$ par 2 moles d'acide $HS(CH_2)_nCOOH$ ou bien d'un
- diacide $HOOC(CH_2)_mCOOH$ par 2 moles d'un thiol-alcool $HS(CH_2)_nOH$

chacun de ces alcools et acides pouvant porter d'autres groupes -OH ou/et -SH. Par exemple un malonate, succinate, hydroxysuccinate, glutarate, adipate, subérate ou sébaçate de di(mercapto-alkyle), l'alkyle étant en $C_1$ à $C_9$.

En tant qu'exemples non limitatifs des composés, répondant à la formule (1) suivant l'invention, peuvent servir les corps suivants:

Rapport SH/C

bis(mercapto-acétate)d'éthylène glycol
$HSCH_2CO-CH_2CH_2-OCCH_2SH$ 1/3
|| ||
O O

bis(mercapto-2 propionate)d'éthylène glycol
$HSCH_2CH_2CO-CH_2CH_2-OCCH_2CH_2SH$ 1/4
|| ||
O O

bis(mercapto-acétate) de glycéryle
$HSCH_2CO-CH_2CHCH_2-OCCH_2SH$ 1/3,5
|| | ||
O OH O

bis(mercapto-acétate)de diéthylène-glycol
$HSCH_2CO-CH_2CH_2-O-CH_2CH_2-OCCH_2SH$ 1/4
|| ||
O O

bis(mercapto-3-butyrate) de butane diol-2,3
$HSCH_2CH_2CH_2CO-CH-CH-OCCH_2CH_2CH_2SH$ 1/6
|| | | ||
O $CH_3$ $CH_3$ O

bis(mercapto-2 propionate) de pentaérythrityle

$CH_2OH$
|
$HSCH_2CH_2CO-CH_2-C$——$CH_2OCCH_2CH_2SH$ 1/5,5
|| | ||
$CH_2OH$ O

Rapport SH/C

tris(mercapto-2 propionate) de mentaérythrityle
CH2OH
|
$HSCH_2CH_2CO-CH_2-C$——$CH_2-OCCH_2CH_2SH$
|| | ||
O $CH_2$ O 1/4,66
|
$OCCH_2CH_2SH$
||
O

bis(mercapto-7 caprylate) de pentanediol 2,4-thiol 3
$HS(CH_2)_7CO-CH-CH-CH-OC(CH_2)_7SH$ 1/7
|| | | | ||
O $CH_3$ SH $CH_3$ O

malonate de di(mercapto-3 propyle)
$HSCH_2CH_2CH_2-OC-CH_2-CO-CH_2CH_2CH_2SH$ 1/4,5
|| ||
O O

succinate de di(mercapto-4 butyle)
$HSCH_2CH_2CH_2CH_2-OC-CH_2CH_2-$
||
O

$CO-CH_2CH_2CH_2CH_2SH$ 1/6
||
O

hydroxy-succinate de di(mercapto-2 éthyle)
$HSCH_2CH_2-OC-CHCH_2-CO-CH_2CH_2SH$ 1/4
|| | ||
O OH O

hydroxy-succinate de di(mercapto-6 hexyle)
$HS(CH_2)_6-OC-CHCH_2-CO-(CH_2)_6SH$ 1/8
|| | ||
O OH O

glutarate de di(mercapto-7 heptyle)
$HS(CH_2)_7-OC-CH_2CH_2CH_2-CO-(CH_2)_7SH$ 1/9,5
|| ||
O O

adipate de di(mercapto-2 éthyle)
$HSCH_2CH_2-OC-(CH_2)_4-CO-CH_2CH_2SH$ 1/5
|| ||
O O

subérate de di(mercapto-2 éthyle)
$HSCH_2CH_2-OC-(CH_2)_6-CO-CH_2CH_2SH$ 1/6
|| ||
O O

sebaçate de di(mercapto-3 propyle)
$HSCH_2CH_2CH_2-OC-(CH_2)_8-CO-CH_2CH_2CH_2SH$
|| ||
O O 1/8

Alors que l'adjonction des composés susindiqués permet de stériliser des objets en chlorure de polyvinyle avec des doses de rayons gamma dépassant 2 Mrads, on ne peut guère dépasser 0,5 Mrad avec du stéarate de mercapto-2 éthyle (rapport SH/C = 1/20), composé qui – d'après la publication du brevet français n° 2 434 835 – constitue un excellent stabilisant vis-à-vis de la chaleur, lorsqu'il est utilisé conjointement avec des organo-stanniques.

Les proportions des esters mercaptans, suivant l'invention, à ajouter au polymère, devant être soumis au rayonnement ionisant, sont généralement du même ordre que celle du stabilisant stannique ou antimoneux; elles se rangent de préférence entre 0,1 et 6% en poids, suivant la nature du polymère et celle du ou des mercaptans choisis, ainsi que selon la dose de rayonnement à utiliser. Dans ces limites, l'effet protecteur augmente avec la teneur en l'ester mercaptan. Pratiquement, les proportions recommandables sont de 0,5 à 5% et surtout de 2 à 4% du poids du polymère.

L'incorporation de ces adjuvants a lieu par malaxage à la température de fort ramollissement de la résine à traiter. Les compositions selon l'invention peuvent contenir également d'autres ingrédients usuels tels que, par exemple, des agents facilitant la mise en œuvre, des agents renforçants, des pigments, des lubrifiants ou encore des stabilisants thermiques auxiliaires des polymères de chlorure de vinyle.

L'invention est illustrée non limitativement par les exemples donnés ci-après.

Exemples 1 à 6

Des essais d'irradiation avec des rayons gamma, émis par une source au cobalt 60, sont effectués sur une composition de chlorure de polyvinyle du type prévu pour le moulage de corps creux, notamment récipients, films et feuilles transparentes ou opaques. A cette composition, on incorpore différents esters mercaptans, avant l'irradiation, pour voir comment se comporteraient des flacons en cette matière, stérilisés aux rayons gamma. La résine est un chlorure de polyvinyle (PVC) d'indice de fluidité K=57, additionné des ingrédients suivants, selon la technique connue.

Pour 100 parties en poids de PVC elle contient:

0,9 partie d'additif de mise en œuvre, un polyalkylacrylate vendu sous la dénomination «Paraloïd K 120 N» par la Société Roehm & Haas;

0,7 partie d'anticollant, copolymère styrène-alkylacrylate, désigné par «Paraloïd K 175» du même producteur;

10 parties de renforçateur antichoc, terpolymère méthacrylate-butadiène-styrène, connu sous le nom de «Kane Ace – B 28 A» (Société Kanegafuchi au Japon);

1,5 parties de stabilisant à la chaleur:
di-n.octyl di(mercapto-acétate d'iso-octyl)étain
$(n.C_8H_{17})_2Sn(SCH_2-CO-i.C_8H_{17})_2$
||
O

3 parties de co-stabilisant à la chaleur, le bis-β aminocrotanate de thio-éthylène glycol
$CH_3C=CH-CS-CH_2CH_2-OC-CH=CCH_3$
| || || |
$NH_2$ O O $NH_2$

correspondant au produit commercial «Irgastab A 70» de la Société Ciba-Geigy.

1,2 parties de lubrifiant, le monostéarate de glycéryle. La composition est malaxée dans un mélangeur rapide Papenmeier jusqu'à ce que la température atteigne 100°C. A partir du mélange homogène, ainsi obtenu, on produit une série de plaquettes de 50×40 mm aux épaisseurs en cascade de 4 mm, 2 mm et 1 mm, par injection, au moyen de la machine Negri-Bossi type V7-9 F. AS. A d'autres portions, de la même composition, on incorpore, dans le mélangeur Papenmeier, les esters mercaptans suivant l'invention, et l'on prépare, pour chacun de ces esters, une série de plaquettes d'essai des dimensions susindiquées.

Les plaquettes sont ensuite soumises à l'action des radiations gamma fournies par une source de cobalt 60. Pour chaque série, les irradiations sont effectuées avec les doses de 0,46, 0,90, et 2,76 Mégarads.

Toutes les plaquettes étant au départ pratiquement incolores, on observe les colorations après chaque irradiation.

Le tableau ci-après récapitule les résultats de ces observations: les intensités de la coloration (jaunâtre ou rougeâtre) sont notées par des signes – ou +;

– signifie que l'échantillon est resté pratiquement incolore;

\+ indique une faible coloration
++ indique une coloration nette;
+++ indique une coloration forte;
++++ indique une coloration très forte.

Tableau I

| Exemple | Ester mercaptan ajouté et son % du PVC | | Dose d'irradiation en Mégarads 0,46 | 0,90 | 2,76 |
|---|---|---|---|---|---|
| | | | Coloration | | |
| 1 | aucun | | ++ | +++ | ++++ |
| 2 | Bis(mercapto-acétate) de glycéryle | 3% | – | – | – |
| 3 | Hydroxy-succinate de di-(mercapto-2-éthyle) | 2% | – | – | ++ |
| 4 | Hydroxy-succinate de di-(mercapto-2-éthyle) | 3% | – | – | + |
| 5 | Adipate de di (mercapto-2 éthyle) | 3% | – | – | ++ |
| 6 | Stéarate de mercapto-2 éthyle | 3% | + | ++ | +++ |

La comparaison des exemples 2 à 5 avec 1 et 6 montre une amélioration considérable de la tenue aux rayons ionisants, grâce à l'incorporation des esters mercaptans spéciaux, suivant l'invention; elle prouve en même temps que les groupes thio-, présents dans l'organostannique et dans l'amino-crotonate de la composition traitée, pas plus que le groupe mercapto du stéréate (ex. 6) n'inhibent l'altération par les rayons gamma; bien que le stéréate améliore légèrement la tenue de la résine, il est pratiquement inefficace à partir de la dose de 0,46 Mégarad.

Par contre, les esters mercaptans suivant l'invention permettent tous des doses au-dessus de 0,9 Mégarad, qui suffisent généralement. Le bis(mercapto-acétate) de glycéryle (ex. 2) rend même possible l'application de la dose maximale de 2,76 Mégarads, ce qui est tout à fait remarquable. L'exemple 4 montre qu'avec l'hydroxysuccinate de di(mercapto-2 éthyle) on peut aller jusqu'à des doses très voisines de 2,76.

Exemples 7 à 12

Le même mode opératoire que dans les exemples précédents est appliqué au même chlorure de polyvinyle auquel on a incorporé 1,8% en poids de di-n.octyl di(mercapto-acétate d'isooctyl) étain, en tant que stabilisant à la chaleur, mais sans addition d'anticollant, de renforçateur antichoc, ni d'additif de mise en œuvre. Dans les exemples 8 à 12 on a ajouté, en outre les mêmes proportions des mêmes mercaptans que respectivement dans les exemples 2 à 6.

Après les irradiations aux rayons gamma on a trouvé pour les échantillons 7 à 12 les mêmes résultats, obtenus respectivement aux exemples 1 à 6.

Exemples 13 à 18

Les essais des exemples 7 à 12, sans autres adjuvants que le composé stannique et le mercaptan, sont répétés, mais le stabilisant stannique employé est le maléate de dibutyl-étain.

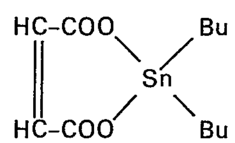

à raison de 2% à la place de celui des exemples précédents. Dans l'exemple 13 on n'ajoute aucun autre additif, tandis que dans les exemples 14 à 18 on introduit 2,7% d'un mercaptan dont la composition est précisée dans le Tableau II de résultats, ci-après.

Tableau II

| Exemple Nº | Ester mercaptan ajouté (2,7%) | Dose d'irradiation en Mégarads 0,46 | 0,90 | 2,76 |
|---|---|---|---|---|
| | | Coloration | | |
| 13 | aucun | ++ | +++ | ++++ |
| 14 | Bis(mercapto-acétate) d'éthylène glycol | – | – | + |
| 15 | Malonate de di (mercapto-3 propyle) | – | – | – |

Tableau II

| Exemple N° | Ester mercaptan ajouté (2,7%) | Dose d'irradiation en Mégarads 0,46 Coloration | 0,90 | 2,76 |
|---|---|---|---|---|
| 16 | Succinate de di (mercapto-4 butyle) | – | – | ++ |
| 17 | Mercapto-6 caproate de méthyle | – | – | + |
| 18 | Mercapto-stéarate d'éthyle | + | ++ | +++ |

On voit que les esters mercaptans N° 14 à 17, dans lesquels le rapport molaire C/SH est compris entre 3 et 10, apportent une bonne protection contre les effets des rayons $\gamma$; par contre le mercapto-stéarate d'éthyle (C/SH = 20), connu comme excellent stabilisant vis-à-vis de la chaleur, donne des mauvais résultats en présence des rayons gamma.

Exemples 19–24

Dans une série de mélanges de chlorure de polyvinyle avec 1,7% de composé stannique et 2,7% de bis(mercapto-acétate) de glycéryle, sans autres adjuvants, on fait varier la nature du composé stannique; les compositions obtenues sont soumises à l'irradiation, comme dans les exemples précédents.
Les stabilisants à l'étain essayés sont:

Exemple
19 - Di-laurate de dibutyl-étain
20 - Diméthyl di(mercapto-acétate de myristyle) étain
21 - Dibutyl di(stéarate de mercapto-2 éthyle) étain
22 - Monobutyl tri(mercapto-oléate d'éthyle) étain
23 - Monobutyl mercapto-acétate d'isooctyl sulfure d'étain
24 - Composé $BuO(SnBu_2O)_2Bu$

Dans tous les cas, la présence de bis(mercapto-acétate de glycéryle améliore la tenue aux rayons $\gamma$ au même degré que dans les exemples 2 à 5 (Tableau I plus haut).

Exemple 25

Dans l'exemple 8, où l'ester mercaptan ajouté est le bis(mercapto-acétate) de glycéryle (comme dans l'exemple 2), on remplace le stabilisant stannique par la même proportion de n.-octyl di(mercapto-acétate d'isooctyl) antimoine.

Les colorations après irradiation sont semblables à celles de l'exemple 2.

Exemple 26

On opère comme dans l'exemple 25, mais le stabilisant thermique à l'antimoine est le tri(mercapto-acétate d'isooctyl) antimoine. L'amélioration de la tenue aux rayons $\gamma$, apportée par le bis(mercapto-acétate) de glycéryle, est semblable à celle de l'exemple 2.

**Revendications**

1. Procédé de stérilisation, par rayonnement ionisant de haute énergie, d'objets en polymère halogéno vinylique ou vinylidénique, ou de produits dont l'emballage contient un tel polymère, ce polymère étant additionné d'un stabilisant à la chaleur, notamment d'un composé organo-stannique ou/et organo-antimoneux, et d'au moins un ester porteur d'une fonction mercaptan, caractérisé en ce que cet ester possède 1 groupe -SH pour 3 à 10 atomes de carbone et qu'il est incorporé dans le polymère avant son irradiation.

2. Procédé suivant la revendication 1, dans lequel l'irradiation est effectuée avec une dose de 0,5 à 2,8 Mégarads.

3. Procédé suivant la revendication 1 ou 2, caractérisé en ce que l'ester mercaptan incorporé est un dithiol, dont les deux groupes -SH se trouvent aux bouts opposés de la chaîne formant sa molécule.

4. Procédé suivant une des revendications 1 à 3, caractérisé en ce que l'ester mercaptan incorporé présente la structure $HS\ (CH_2)_n\text{-}Z\text{-}(CH_2)_m\text{-}Z(CH_2)_nSH$ où Z désigne le groupe carboxy

-C-O-
||
O

dont l'atome de C est lié à un carbone des chaînes $-(CH_2)_n-$ ou de la chaîne $-(CH_2)_m-$; n est un nombre entier de 1 à 9 et m de 1 à 18, la somme 2n+m étant d'au moins 4.

5. Procédé suivant la revendication 4, caractérisé en ce que l'ester mercaptan est un diester de 2 moles d'un mercapto-acide carboxylique avec 1 mole de polyol, notamment un glycol ou le glycérol pouvant porter de substituants, l'ester étant en particulier le bis(mercapto-acétate)-1,3 de glycéryle.

6. Procédé suivant la revendication 5, caractérisé en ce que l'ester mercaptan est un diester de 1 mole d'un diacide carboxylique avec 2 moles d'un thiol-alcool.

7. Procédé suivant la revendication 6, caractérisé en ce que l'ester mercaptan est un malonate, succinate, hydroxysuccinate, glutarate, adipate, subérate ou sébaçate de di(mercapto-alkyle), l'alkyle étant en $C_1$ à $C_9$.

8. Procédé suivant une des revendications précédentes, appliqué à du chlorure de polyvinyle, caractérisé en ce qu'on incorpore à celui-ci 0,1 à 6% en poids de dit mercapto-ester renfermant 1 groupe SH par 3 à 10 atomes de carbone, le rayonnement ionisant étant constitué par des rayons gamma.

9. Object en matière plastique, halogéno vinylique ou vinylidénique, stabilisée à la chaleur par un composé d'organo-étain et/ou d'organo-antimoine, pouvant renfermer un costabilisant, notamment un thio- ou mercapto-composé, cet objet ayant subi l'irradiation par un rayonnement ionisant de haute énergie, caractérisé en ce qu'il contient un ester mercaptan porteur d'un ou de plusieurs groupes -SH, à raison d'un SH pour 3 à 10 atomes de carbone, et que son polymère est pratiquement incolore.

10. Objet suivant la revendication 9, dont le polymère est du chlorure de polyvinyle, stérilisé au moyen de rayons gamma, caractérisé en ce que ledit ester mercaptan porte un groupe -SH à chacun des bouts de la chaîne constituant sa molécule et se trouve à la proportion de 0,1 à 6% du poids du polymère.

**Patentansprüche**

1. Verfahren zur Sterilisierung von Halogen-vinyl- oder -vinyliden-Polymer-Gegenständen oder von Produkten, deren Verpackung ein derartiges Polymeres enthält, durch ionisierende Strahlung mit hoher Energie, wobei das Polymere mit einem Wärmestabilisierungsmittel, insbesondere einer Organo-Zinn-IV- und/oder einer Organo-Antimon-III-Verbindung und mindestens einem Ester, der eine Mercaptanfunktion trägt, versetzt ist, dadurch gekennzeichnet, dass der Ester 1 Gruppe -SH pro 3 bis 10 Kohlenstoffatome trägt und dass er in das Polymere vor dessen Bestrahlung eingearbeitet wird.

2. Verfahren nach Anspruch 1, bei dem die Bestrahlung mit einer Dosierung von 0,5 bis 2,8 Megarad durchgeführt wird.

3. Verfahren nach Anspruch 1 oder 2, dadurch gekennzeichnet, dass der eingearbeitete Mercaptanester ein Dithiol ist, dessen beide Gruppen -SH sich an den entgegengesetzten Enden der sein Molekül bildenden Kette befinden.

4. Verfahren nach einem der Ansprüche 1 bis 3, dadurch gekennzeichnet, dass der eingearbeitete Mercaptanester die Struktur $HS(CH_2)_n$-Z-$(CH_2)_m$-Z$(CH_2)_n$SH aufweist, worin Z die Carboxygruppe

-C-O-
||
O

bedeutet, deren C-Atom an einen Kohlenstoff der Ketten $-(CH_2)_n-$ oder der Kette $-(CH_2)_m-$ gebunden ist; n eine ganze Zahl von 1 bis 9 und m von 1 bis 18 ist, wobei die Summe 2n+m mindestens 4 beträgt.

5. Verfahren nach Anspruch 4, dadurch gekennzeichnet, dass der Mercaptanester ein Diester von 2 Mol einer Mercaptocarbonsäure mit 1 Mol Polyol, insbesondere einem Glykol oder Glycerin, das Substituenten tragen kann, ist, wobei der Ester insbesondere das Glyceryl-1,3-bis(mercapto-acetat) ist.

6. Verfahren nach Anspruch 5, dadurch gekennzeichnet, dass der Mercaptanester ein Diester von 1 Mol einer Dicarbonsäure mit 2 Mol eines Thiolalkohols ist.

7. Verfahren nach Anspruch 6, dadurch gekennzeichnet, dass der Mercaptanester ein Di(mercapto-alkyl)-malonat, -succinat, -hydroxysuccinat, -glutarat, -adipat, -suberat oder -sebacat ist, dessen Alkyl $C_1$-$C_9$ aufweist.

8. Verfahren nach einem der vorhergehenden Ansprüche, angewendet auf Polyvinylchlorid, dadurch gekennzeichnet, dass man in dieses 0,1 bis 6 Gew.-% des genannten Mercaptoesters mit 1 Gruppe -SH pro 3 bis 10 Kohlenstoffatome einarbeitet und die ionisierende Strahlung aus Gammastrahlen besteht.

9. Gegenstand aus Halogen-vinyl- oder -vinyliden-Kunststoffmaterial, stabilisiert gegen die Wärme mittels einer Organo-Zinn- und/oder Organo-Antimon-Verbindung, der ein Costabilisierungsmittel, insbesondere eine Thio- oder Mercapto-Verbindung enthalten kann, wobei dieser Gegenstand einer Bestrahlung mit ionisierender Strahlung hoher Energie unterzogen wurde, dadurch gekennzeichnet, dass er einen Mercaptanester enthält, der eine oder mehrere Gruppen -SH in einem Anteil von einem SH pro 3 bis 10 Kohlenstoffatome enthält, und dass sein Polymeres praktisch farblos ist.

10. Gegenstand nach Anspruch 9, dessen Polymeres Polyvinylchlorid, sterilisiert mittels Gammastrahlen, ist, dadurch gekennzeichnet, dass der genannte Mercaptanester eine Gruppe -SH an jedem Ende der sein Molekül bildenden Kette trägt und im Anteil von 0,1 bis 6% des Gewichts des Polymeren vorhanden ist.

**Claims**

1. Process of sterilization by high-energy ionizing radiation of articles comprising halogenovinyl or vinylidene polymers or products in packaging which contains such a polymer, the polymer including a heat stabilizer, in particular an organotin and/or organo-antimony compound, and at least one ester carrying a mercaptan function, characterized in that the ester has 1 -SH group per 3 to 10 carbon atoms and is incorporated into the polymer before its irradiation.

2. Process according to claim 1, in which the irradiation is effected with a dose of 0.5 to 2.8 megarads.

3. Process according to claim 1 or 2, characterized in that the mercaptan ester incorporated is a dithiol, the two -SH groups of which are located at the opposite ends of the chain forming its molecule.

4. Process according to any of claims 1 to 3, characterized in that the mercaptan ester incorporated has the structure $HS(CH_2)_n$-Z-$(CH_2)_m$-

$Z(CH_2)_nSH$, where Z designates the carboxy group

$$\begin{array}{l}-C-O-\\ \;\;\| \\ \;\;O\end{array}$$

the C atom of which is connected to a carbon atom of the $-(CH_2)_n-$chains or of the $-(CH_2)_m-$chain, n is an integral number from 1 to 9 and m from 1 to 18, the sum 2n+m being at least 4.

5. Process according to claim 4, characterized in that the mercaptan ester is a diester of 2 moles of a mercapto-carboxylic acid with 1 mole of a polyol, particularly a glycol or glycerol, optionally carrying substituents, the ester being in particular glyceryl-1,3-bis (mercapto-acetate).

6. Process according to claim 5, characterized in that the mercaptan ester is a diester of 1 mole of a carboxylic diacid with 2 moles of a thiol-alcohol.

7. Process according to claim 6, characterized in that the mercaptan ester is a di (mercapto-alkyl) malonate, succinate, hydroxysuccinate, glutarate, adipate, suberate or sebacate, the alkyl containing $C_1$ to $C_9$.

8. Process according to any of the preceding claims, applied to polyvinylchloride, characterized in that 0.1% to 6% by weight of the mercapto-ester containing 1 SH group per 3 to 10 atoms of carbon is incorporated, the ionizing radiation comprising gamma rays.

9. An article of halogeno-vinyl or vinylidene plastics material, stabilized to heat by an organo-tin and/or organo-antimony compound, which can contain a co-stabilizer, particularly a thio or mercapto compound, the article having undergone irradiation by high-energy ionizing radiation, characterized in that it contains a mercaptan ester carrying one or more -SH groups, in the ratio of 1 SH per 3 to 10 atoms or carbon, and that its polymer is substantially colourless.

10. Article according to claim 9, the polymer of which is polyvinylchloride, sterilized by means of gamma rays, characterized in that the mercaptan ester carries an SH group at each of the ends of the chain constituting its molecule and is present in an amount of 0.1 to 6% by weight of the polymer.